# EUROPEAN PATENT SPECIFICATION

(11) **EP 2 259 815 B1**
(45) Date of publication and mention of the grant of the patent: **26.06.2019**
(21) Application number: 09707582.4
(22) Date of filing: 06.02.2009
(51) Int. Cl.: A61M 5/158, A61M 5/142

(54) **ASSEMBLY COMPRISING INSERTER, CANNULA PART AND BASE PART**
ANORDNUNG MIT EINFÜHRINSTRUMENT, KANÜLENTEIL UND BODENTEIL
ENSEMBLE COMPRENANT UN DISPOSITIF D'INSERTION, UNE PARTIE CANULE ET UNE PARTIE BASE

(30) Priority: 08.02.2008 DK 200800185; 08.02.2008 US 27125
(43) Date of publication of application: 15.12.2010
(73) Proprietor: Unomedical A/S, 3460 Birkerød (DK)
(72) Inventor: HØRDUM, Elo, Lau, DK-2970 Hørsholm (DK); GYRN, Steffen, DK-4100 Ringsted (DK); HICKMOTT, Richard, Morgan, DK-2100 København Ø (DK); MORTON, Alistair, David, DK-2770 Kastrup (DK)
(74) Representative: Campabadal i Monfà, Gemma
(86) International application number: PCT/EP2009/051380
(87) International publication number: WO 2009/098291

(56) References cited:
- WO-A1-2008/014791
- US-A- 5 267 963
- US-A1- 2002 077 599
- US-A1- 2004 158 207
- US-B1- 6 620 140

## Description

### The technical field

The invention relates to an assembly for providing continuous administration of a therapeutically working substance, such as insulin, or continuous reading of a component which assembly comprises a base part, which is to be fastened to a patients skin, to which an insertion device and delivery means are fastened. Delivery means normally comprises a reservoir and e.g. a pump, and the insertion device comprises means for inserting a penetrating member e.g. in the form of a cannula or a sensor.

### Prior art

WO 2007/071258 describes a medical device for delivering fluid comprising an injection part and a fluid delivery part where the fluid delivery part and the injection part can be separated and rejoined. The fluid delivery part comprises a reservoir, means for transport of liquid e.g. in form of a pump and a house in which the active units of the delivery part is placed. The injection part comprises: a base plate, a cannula part comprising a body with a through going opening provided with a cannula extending past the proximal side of the base plate and means for fixation of the base plate to the skin of the user e.g. in the form of a mounting pad. The delivery part and the injection part is assembled through a connector comprising a fluid path leading fluid from the reservoir to the through-going opening in the cannula part which fluid path comprises means for blocking access to the injection part when the connector is disconnected from the delivery part and/or the injection part. The embodiments illustrated in this document are quite complex and not easy to manufacture.

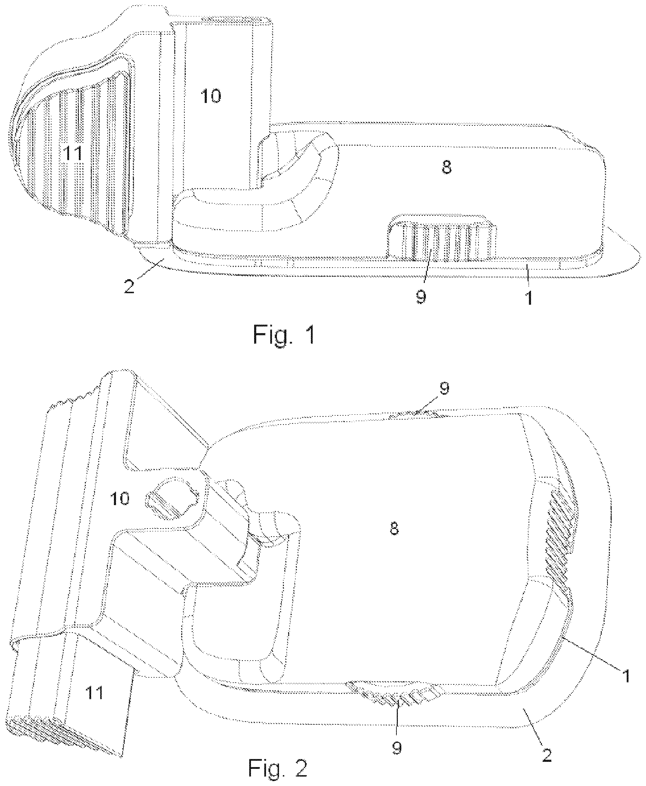

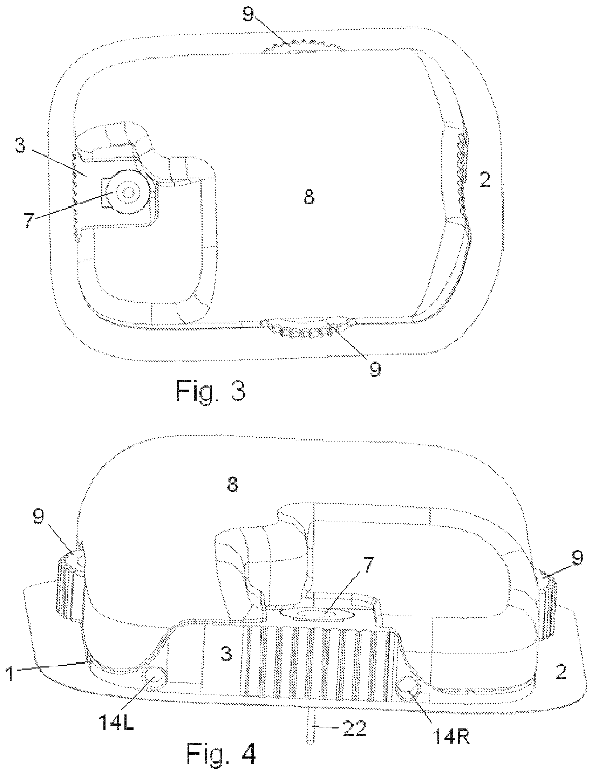

US 2007/0191772 discloses an infusion set assembly comprising an infusion device provided with a manual inserter i.e. the insertion needle is inserted by pushing the insertion needle into the patients skin. The infusion set assembly is desirably delivered to the user in a sterile package in a pre-assembled configuration as shown in fig. 1 and 3. A base part (15) named cannula housing includes a pair of pivot holes (26) configured to snap-fittingly reception of pivot pins (25) placed on the penetrating member (16) which is named septum-housing, the pivot holes (26) are desirably through holes opening both inwardly and outwardly, and the pivot pins (25) are located at the end of flexible arms (24) configured to flex inward. When the pivot pins (25) are placed in the pivot holes (26) the penetrating member (16) is retained in the base part (15) and can pivot between an upright and a horizontal position. An insertion guide housing (68) guides the movement of an insertion needle assembly (38) comprising an insertion needle, the insertion guide housing (68) rests on the top surface of the base part (15) and is initially attached to the base part by flexible barbs (79) which engage the outward portions of pivot holes (26). When pivot pins (25) engage pivot holes (26), pivot pins (25) extend through holes (26) such that the barbs (79) on the insertion guide housing (68) are pushed outward by pivot pins (25) thereby unlatching the insertion guide housing (68) from the base part. According to this disclosure the disengagement of the inserter (38, 68) is caused by the movement and position of the penetrating member (16), this is not desirably as this increases the risk of incorrect positioning of the penetrating member (16) as the pivot pins which are parts of the penetrating member might not end in the desired position in the pivot holes. If the assembly malfunctions when positioning the penetrating member it will be necessary to remove the infusion assembly from the patients skin and try again with a new assembly.

US2004158207 discloses a device for inserting a cannula into tissue, including a cannula a protective element which can accommodate said cannula, an operating element for moving the cannula out of the protective element, and a holder fixedly connected to the cannula. The document also discloses a system for connecting a liquid supply to the cannula.

### The invention

The object of the invention is to provide an assembly which solves the problem of positioning the penetrating member in connection with the inserter and of positioning the inserter in connection with the base part and thereby positioning the penetrating member in relation to the patient. If these actions are not performed correct, they each present a risk of contamination of the penetrating member or misplacement of the penetrating member and of incorrect positioning of the inserter and consequently infection at the insertion point or misplacement of the penetrating member can take place.

It is also an object of the invention to eliminate some of the problems experienced with the infusions assembly described in the prior art e.g. the penetrating part is not involved in the releasing of the inserter as this increases the risk of malfunction.

The problems are solved with a device according to claim 1 which device relates to an assembly comprising:
- an inserter for insertion of a penetrating member into a patient,
- a penetrating member attached to the inserter before use which penetrating member is provided with a through going opening forming a fluid connection between delivery means and a patient when the penetrating member is inserted,
- a base part comprises a position shaped to receive the penetrating member where the penetrating member can be attached to the base part during use and fastening means which can provide for the base part to be attached to the patient,
where the inserter comprises inserter attachment means locking the inserter to the base part before and during insertion of the penetrating member. which inserter attachment means can be unlocked so that the inserter can be removed from the base part after insertion of the penetrating member, the inserter attachment means comprises at least one protruding part and at least one corresponding opening. Either the at least one protruding part or the at least one corresponding opening part is positioned on a surface of the base part and the part corresponding to this protrusion or opening is positioned on a surface of the inserter. Either the penetrating member or the base part or both is/are provided with sealing means forming a fluid tight connection between the base part and the penetrating member and the base part is further provided with sealing means forming a fluid connection to delivery means.

When the inserter attachment means are positioned respectively on a surface of the inserter and on a surface of the base part the movement of the penetrating part from the start position inside the inserter to the end position in the base part will not be influenced by the disengagement of the inserter.

Normally there is more than one corresponding pair of protrusion and opening and then all attachment means on the inserter and respectively on the base part are facing in the same direction i.e. when the one or more corresponding pairs of protruding parts and openings are positioned only on one side of the base part, then the inserter can be disengaged from the base part by moving the whole inserter in one direction.

According to one embodiment the inserter is released from the base part i.e. the inserter attachment means are disengaged, by the interaction between a moving part of the inserter which moving part guides the insertion of the penetrating member from a first to a final position and a stationary part of the base part during insertion. The moving part of the inserter can move forward in a direction which is approximately perpendicular i.e. 90°±45°, normally 90°±15° to the direction of insertion of the penetrating member.

According to one of the embodiments according to the invention a further problem solved with such an assembly is that if it is provided with an automatic release mechanism the risk of displacing, e.g. partly or fully pulling out the penetrating member, is significantly reduced when removing the inserter from the base part.

An assembly according to claim 1 comprising an inserter for insertion of a penetrating member into a patient and a base part. The base part is provided with fastening means which can provide for the base part to be attached to the patient and the penetrating member is provided with a through going opening forming a fluid connection between delivery means and a patient when the penetrating member Is inserted. The device is characterized in that the inserter comprises attachment means locking the inserter to the base part before and during insertion of the penetrating member.

Accordina to one embodiment the attachment means can be unlocked so that the inserter can be removed from the base part after insertion of the penetrating member.

According to one embodiment the inserter is released from the base part by the interaction between a moving part of the inserter and a stationary part of the base part during insertion. "A stationary part" means that the part does not move in the same forward direction as the moving part, the stationary part might move in a direction perpendicular to the forward direction of the moving part. The interaction is to be understood as a physical contact which is reached at a specific time and place during the forward movement of the moving part, between a part of the moving part and a part of the inserter housing or a part stationary in relation to the inserter housing.

According to one embodiment the penetrating member is attached by a connection to the base part before and during insertion. Normally the connection will provide a permanent attachment of the penetrating member to the base part, i.e. the penetrating member is also attached to the base part after insertion. The connection can comprise a flexible material. This flexible material could e.g. be in the form of a tube providing a fluid path between the penetrating member and the base part. The word "flexible" can both mean that the material is elastic and that the material is soft and bendable.

According to another embodiment the inserter is released from the base part by applying a force to the inserter or a part of the inserter in a direction different from the direction of insertion of the penetrating member. Removal or detachment of the inserter can according to some embodiments not take place before the penetrating member is fully inserted in the base part i.e. the penetrating member is before insertion positioned partly inserted into the base part. The force can be applied in a direction which is approximately perpendicular i.e. 90°±45°, normally 90°±15° to the direction of insertion of the penetrating member.

According to a third embodiment the release of the inserter from the base part is at least partly provided by the release of a force in direction towards the base part. This force presses or pushes the base part toward the skin of the patient in order to prevent release of the base part and the penetrating member from the patient. The force can be provided by a spring unit exercising a force directed to a proximal surface of the inserter housing and a distal surface of the base part. That the surface is proximal means that it is turned toward the patient after insertion of the penetrating member and that the surface is distal means that it is turned away from the patient after insertion of the penetrating member.

The spring can be a leaf spring which at one end is fastened unreleasably to a part of the inserter and at the other end will touch the upper surface of the base part before the inserter is released from the base part.

According to a fourth embodiment the attachment means of the inserter comprise at least one protruding part corresponding to an opening in the base part. That the protruding part corresponds to the opening means that the protruding part fits closely into the opening, further the protruding parts normally have either a round or polygonal cross-section. The at least one protruding part can be inserted into the corresponding opening in a direction approximately parallel to the upper surface of the base part. That the direction is approximately parallel means that the insertion direction of the protruding part can deviate between 0° and 10° from the plane formed by the upper surface of the base part.

According to a fifth embodiment the attachment means comprise at least one hook formed by a part of the housing of the inserter. The at least one hook can be turned inward i.e. the hook is placed on an inner surface of the housing of the inserter and turned toward the centre of the housing. Further the at least one hook can face at least two protruding parts which are placed on a surface of the housing opposite the at least one hook. Also the at least one hook can face two protruding parts while at the same time the at least one hook is placed between the two protruding parts. When a single or double hook is placed between the opposite positioned protruding parts the inserter is fastened in a very stable triangular positioning.

According to one embodiment the penetrating member is provided with attachment means assuring that the penetrating member is unreleasably attached to the base part after insertion.

According to one embodiment the penetrating member is provided with sealing means along the edge of an opening forming the fluid connection to the base part. If the opening is round the sealing will have the form of an O-ring.

According with a sixth embodiment the base part is provided with a longitudinal opening extending from the upper surface of the connection part of the base part to the lower surface of the connection part of the base part, which opening corresponds to the profile of at least the non-penetrating end of the penetrating member. After insertion the penetrating part of the penetrating member is inserted below the patient's skin. "Corresponds" normally means that the two parts have a close fit where opening and the inserted member have the same shape. An outlet of a fluid path can open into the wall of the opening and when the penetrating member is inserted, an inlet to a fluid path of the penetrating member corresponds to the outlet of the fluid path.

According to one embodiment at least a part of the base part comprises a hard material provided with an adhesive surface on the proximal side of the device, i.e. the base part can be adhered to a patient's skin. The proximal side is the side of the device which is closest to the patient's skin, that the material is hard means that it can not be penetrated by an insertion needle and the material can not be compressed or expanded but might be bended if the thickness of the material allows it. The hard material can be provided with an opening through which the penetrating member can be inserted and the hard material can e.g. be a molded plastic material such as polypropylene.

According to one embodiment one penetrating member comprises a cannula part having a body made of a hard material having a soft or cutting cannula attached unreleasably. A cannula is a part providing a fluid path way right through a patient's skin; normally it is a hollow needle. If the cannula is soft it has to be inserted with a separate insertion needle and if the cannula is cutting it is itself able to cut through the patient's skin and need not be inserted with a separate insertion needle. Normally medication is transported to the patient via the fluid path way or blood or other liquid is transported from the patient. Preferably the body of the cannula part has an inner opening from which inner opening fluid is led to the cannula and this inner opening has at least one inlet through which fluid or a needle can enter the inner opening. The inner opening can have two inlets through which fluid or a needle can enter the inner opening.

According to one embodiment at least one penetrating member comprises a sensor part. A sensor part provides a contact right through the patient's skin, nothing physical needs to be transported normally only signals are transported between the end of the sensor which is in contact with the patient's blood and the end of the sensor which is in contact with a reader registering the signals.

According to one embodiment the assembly is delivered to the user in a sterile state where the penetrating member is in a retracted position in the inserter and the inserter is attached to the base part. "Sterile state" is a protected uncontaminated state in which the assembly can be stored ready for use. When the user receives the assembly the user will just have to unpack or otherwise reveal it and put it to use after e.g. first having cleaned the position on the patients skin where the penetrating member is to be inserted.

Embodiments of the invention will now be described with reference to the figures in which:
Figure 1 shows a first embodiment of an assembly according to the invention seen from the side where a delivery part and an inserter are connected to a base part.
Figure 2 shows the embodiment of fig. 1 seen from above.
Figure 3 shows the embodiment of fig. 1 and 2 after insertion of the cannula part and removal of the inserter.
Figure 4 shows the embodiment of fig. 1 seen from the end where the inserter was fastened before the penetrating member was inserted.
Figure 5A shows the delivery part separated from the base part seen from below.
Figure 5B shows the delivery part separated from the base part seen from above.
Figure 6 shows a second embodiment of a base part which can be used in an assembly according to the invention.
Figure 7 shows a longitudinal cut through an assembly as shown in fig. 1-5, the cut is placed at the position of one of the fastening means for the inserter.
Figure 8 shows the inserter without being attached to the base part.
Figure 9A-F shows a second embodiment of an inserter and a base part.
Figure 10 shows one embodiment of a penetrating member which can be used with the assembly.
Figure 11 shows a cut-through view of a second embodiment of a penetrating member which can be used with the assembly.
Figure 12 shows a third embodiment of an inserter to be used with the assembly in a state before insertion of a cannula part.
Figure 13 shows the same embodiment of an inserter as fig. 12 after insertion of a cannula part.
Figure 14A-C show how the embodiment of the inserter shown in fig. 12 and 13 are being detached from a base part of an assembly according to the invention.
Figure 15A and 15B shows an embodiment of a moving part to be used with an assembly as shown in fig. 12-14.
Figure 16A and 16B show an embodiment of a base part.
Figure 17A and 17B shows a fourth embodiment of an inserter to be used with the assembly in a state after insertion of a cannula part.
Figure 18A and 18B show the internal parts of the inserter housing of the fourth embodiment of the inserter.
Figure 19 shows an embodiment of a moving part to be used with an assembly as shown in fig. 17-18
Figure 20 schematically shows an embodiment of an assembly according to the invention.

The figs. 1-5 show an assembly comprising an inserter 10, a delivery part 8 and a base part according to the invention. The base part comprises a surface plate 1 attached to a contact surface, the surface plate 1 is normally made of a hard material provided with an adhesive surface on the proximal side of the device. The surface plate 1 according to this embodiment is constructed of a molded plastic material which could e.g. be polypropylene and the contact surface is the proximal side of a mounting pad 2 which mounting pad 2 is unreleasably fastened to the surface plate 1 during manufacturing of the device. "Proximal" means the side or surface closest to the patient when the mounting pad is adhered to the patient, "distal" means the end or surface furthest away from the patient when the device is in a position of use.

Fig. 1 shows the embodiment of the assembly seen from the side and fig. 2 shows the same embodiment seen from above. Fig. 3 shows the same embodiment seen from above but in fig. 3 the penetrating member has been inserted and the inserter has been removed from the assembly. The penetrating member of this embodiment is comprised in a cannula part 7 which is inserted into an opening 12A of a connector part 3 of the base part, this cannula opening 12A provides and opening which extends right through the base part. The cannula part 7 is provided with a penetrating member in the form of a cannula 22 which in this position has penetrated the surface of the skin of the patient during the insertion and is positioned sub- or transcutaneously.

The inserter 10 holds the cannula part 7 before insertion and the insertion is initiated by pushing a handle 11. Fig. 2 shows the direction the handle 11 has to be pushed in order to initiate insertion of the cannula part 7. After insertion a not shown insertion needle can be retracted to the inside of the inserter 10 and the inserter 10 can be removed from the base part, leaving an inserted cannula 22 fastened to the surface plate 1. If the cannula 22 of the cannula part 7 is a hard self penetrating cannula there will be no separate insertion needle and therefore no need to retract the insertion needle. The inserter 10 is attached near to one end of the surface plate 1 which according to one embodiment makes it possible to pivot the inserter 10 in order to disengage the inserter from the base plate after insertion of the cannula 22. Actually the inserter 10 will normally protrude over the patients skin relative to the surface plate 1.

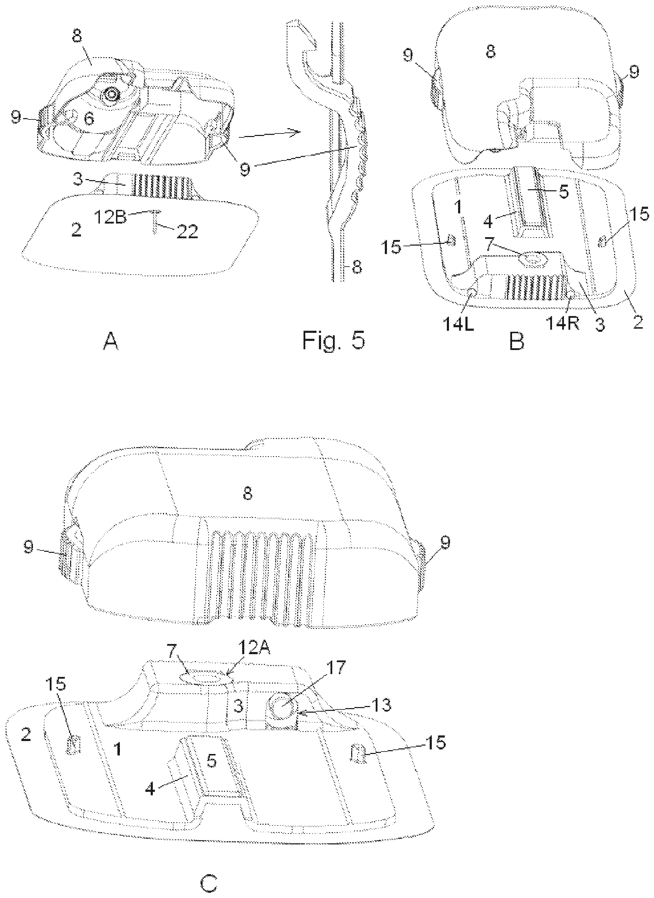

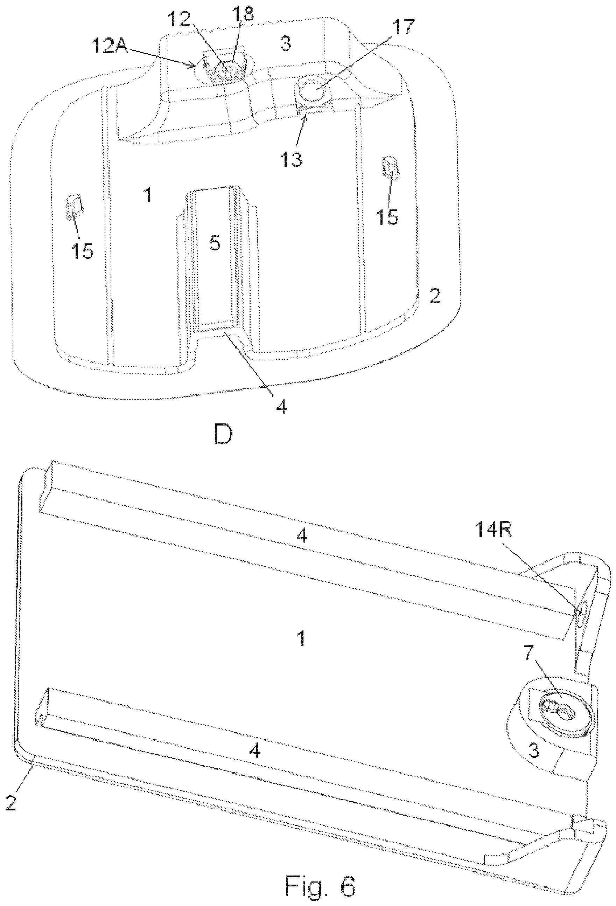

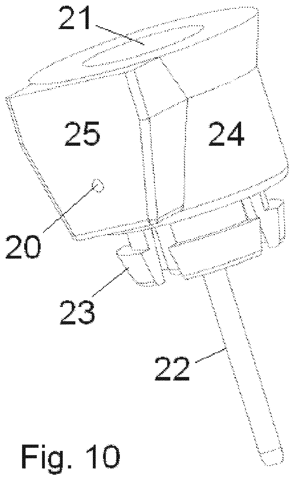

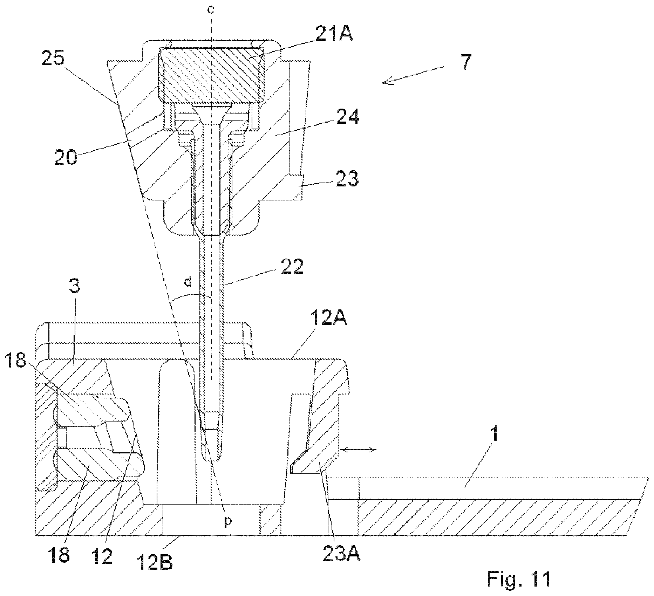

The connector part 3 is kept in position by the surface plate 1. According to one embodiment the surface plate 1 and at least an outer cover of the connector part 3 is simply molded in one piece during manufacturing of the device. The connector part 3 forms a fluid path between e.g. a reservoir 6 of medication or a reservoir for liquid collected from the patient and a cannula part 7. Therefore the connector part 3 is provided with at least two openings, one opening at each end of the fluid path where the first opening 13 is an inlet or outlet opening receiving or delivering fluid to a reservoir 6 and the second opening 12 is an inlet or outlet opening receiving or delivering fluid to a cannula part 7 (see fig. 5C-D). The connection part 3 might be provided with extra openings e.g. for injection of a second medication or nutrient or for letting the fluid in the fluid path get in contact with a sensor. In order to secure a fluid tight connection between the outlet opening 12 in the connection part 3 and the cannula part 7 the outlet opening 12 of the connection part 3 is provided with an elastic sealing 18 around the outlet opening 12. When the cannula part 7 is inserted it will be press fitted into the cannula opening 12 and the elastic sealing 18 will provide a completely fluid tight gasket around the corresponding openings 12 and 20 (see fig. 10 and 11). In order to improved the press-fitting and thereby the fluid tight connection between the cannula part 7 and the outlet of the fluid path, the cannula opening 12A can be provided with a decreasing cross-section in a plane parallel to the cannula 22 when inserted and perpendicular to the surface where the outlet of the fluid path is positioned. The cannula part 7 will have a corresponding decreasing cross-section.

In the following the first opening 13 will be referred to as "inlet" and the second opening 12 will be referred to as "outlet" although the direction of the flow through the fluid path is not significant for the invention.

The connection part 3 is further provided with a cannula opening 12A which accurately fits around a cannula part 7 i.e. the cannula opening 12A has the same shape or profile as the cannula part 7 and is just big enough to let the cannula part 7 pass through and then fit into the opening. When the cannula part 7 is fully inserted into the base part and the patient, then the upper surface i.e. the distal surface of the cannula part 7 is normally at level with or at a lower level than the outer surface of the connection part 3 surrounding the cannula opening 12A.

When the cannula part 7 has been fully inserted into the connection part 3, then an opening in a side surface of the body of the cannula part 7 corresponds to the opening 12 of the fluid path of the connection part 3 and fluid can flow from one part to the other.

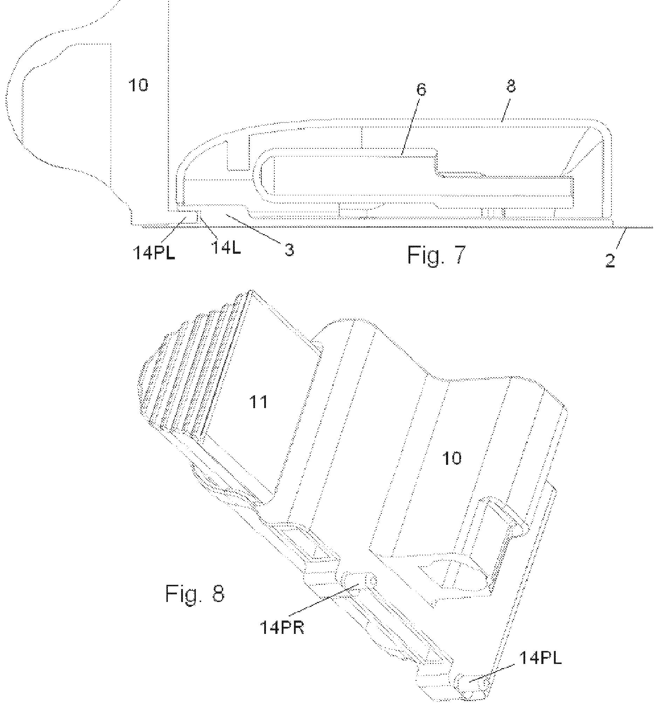

Fig. 4 shows the same embodiment as fig. 3 where the inserter has been removed but fig. 4 shows the device from the end which was covered by the inserter 10 before the inserter was removed. From this end it is possible to see a part of the fastening means 14 which assure attachment of the inserter 10 to the base part before insertion. According to this embodiment the fastening means 14 comprise two openings 14L and 14R in the connector part 3. These openings correspond to two protruding parts 14PL and 14PR (see fig. 7 and 8) which protrude from the side of the inserter housing turned towards the base part and the connector part 3 with the corresponding opening. When the fastening means 14L and 14R on the base part is engaged with the corresponding fastening means 14PL and 14PR on the inserter 10, the inserter 10 is prevented from moving in relation to the base part, at least in the direction perpendicular to the surface plate 1. After insertion of the penetrating member where the penetrating member has been fully inserted into the base part as shown in fig. 4, the inserter 10 can be removed or detached from the base part. When detaching the inserter 10 from the base part, the Inserter 10 is moved in a direction horizontal to the patients skin i.e. the base part is not subjected to a force perpendicular to the patients skin i.e. a force pulling the base part away from the patient.

A skilled person could easily suggest other methods for fastening of the inserter to the base part both mechanical methods and methods comprising adhesive surfaces.

Figs. 5A-D show the base part and the delivery part in a separated position from different angles.

In fig. 5A the two parts are shown from below. This view shows an opening 12B through which the penetrating member 7 can be inserted through the base part and through which opening 12B the cannula 22 extends. From this view it is possible to see how the reservoir 6 can be positioned in the delivery part 8 and to see how two opposite positioned release handles 9 are placed at the edge of the delivery part 8. Further a longitudinal track corresponding to longitudinal raised guiding means 4 on the base part can be seen.

The two release handles 9 are formed as s-shaped bands where one end is fastened hinge-like to the housing of the delivery part 8 and the first curve in the s-shape is slightly extending the outer surface of the housing of the delivery part whereas the second curve is free i.e. not attached to the housing of the delivery part 8 and is provided with a hook-like shape which can fold around a part 15 protruding from the distal surface of the base part. When the delivery part is locked to the base part both release handles 9 are folded round a protruding part 15, when the delivery part 8 is to be removed from the base part, the two opposite release handles 9 are pushed together whereby the hook-like parts of the release handles 9 are released from the protruding parts 15 of the base part, and the delivery part can be moved backwards i.e. in the direction away from the cannula part 7 and removed from the base part in this direction.

In fig. 5B the two parts are shown from above. This view shows how the delivery part 8 of this embodiment can be joined to the base part by pushing the delivery part 8 down toward the guiding means 4 which in this case is a longitudinal raised platform having e.g. a metal lining 5 fastened to the top surface. The delivery part 8 is provided with corresponding means e.g. comprising a track corresponding to the raised platform 4. The corresponding means of the delivery part 8 can slide along the metal lining 5 of the raised platform 4 of the base part in the longitudinal direction. When the delivery part 8 arrives at its working position, the two release handles 9 engage respectively with the two protruding parts 15 protruding from the upper surface of the surface plate 1. When the delivery part 8 is in its working position it is locked in any horizontal direction by the release handles 9. The locking mechanisms make it possible to fasten and release the delivery device from the base part as often as needed i.e. a single-use base part can be combined with a multi-use delivery part.

In fig. 5C the two parts are shown from the end opposite of where the inserter was fastened before insertion of the penetrating member. From this side it is possible to see the inlet opening 13 in the connection part 3 through which e.g. medication from the reservoir 6 can enter, the inlet opening 13 is protected with a membrane to prevent contamination with microorganisms. According to one embodiment the connection part 3 is provided with both a connector needle (not shown as it is placed behind the bubble shaped membrane) and a bubble shaped self closing membrane 17 and the reservoir 6 can be provided with a bubble shaped self closing membrane. Hereby a fluid path is established providing transfer of medication e.g. insulin or nutrients from the reservoir to the connector part 3. As both parts are provided with self closing membranes it will be possible to separate the two units from each other and rejoin them at a later time without the connection part 3 and thereby the patient being contaminated.

Fig. 6 shows a second embodiment of the base part. This embodiment is provided with two guiding means 4 in the form of two right angled profiles shaped as: ┐ ┌, and protruding from the surface plate 1 of the base part. The guiding means 4 correspond to means on a delivery part or a cover which is to be fastened to the base part. Such corresponding means can e.g. be formed as one or more hooks having a profile in the form of ┘ and └.

The fluid path of the connection part 3 is very short compared to the embodiment shown in fig. 1-6 and the inlet of the connection part 3 is placed in a centre position in relation to the guiding means 4 but the inserted cannula part 7 has the same profile as the cannula part 7 fitted to the embodiment of fig. 1-6.

Fig. 7 shows a longitudinal cut through an assembly as shown in fig. 1-5. From this view it is possible to the how the fastening means 14 of respectively the connector part 3 of the base part and the inserter 10 are joined together.

Fig. 8 shows the inserter 10 removed from the rest of the assembly. From this side it is possible to see the fastening means 14PR and 14PL of the inserter.

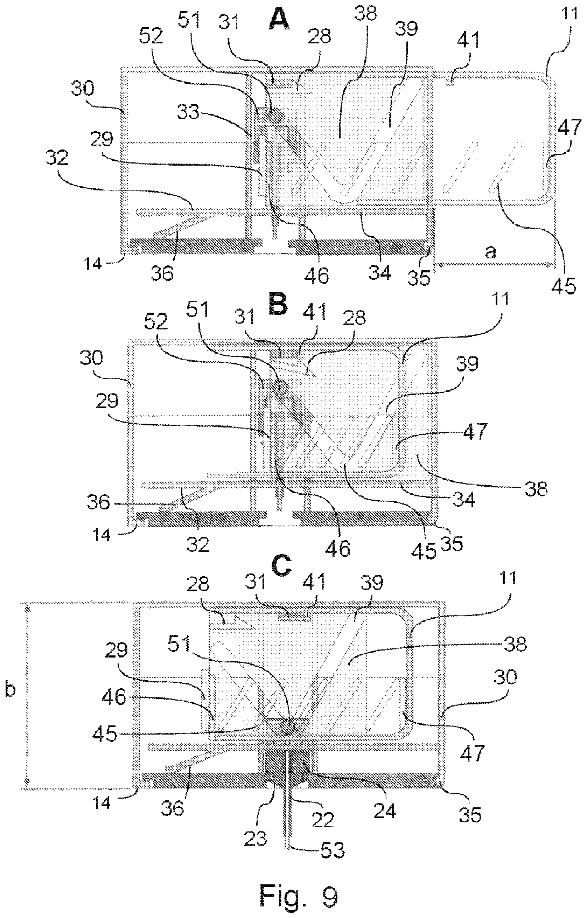

Fig. 9A-9F illustrates attachments means with an automatic release function. The insertion device 1 comprises a housing 30, a base part 100, a moving part 38, an actuator handle 11, and a penetrating member. The penetrating member is similar to the penetrating member described in figure 10. For illustrative purposes means such as the moving part 38 are represented in a semi-transparent fashion.

The housing 30 comprises guiding means 32 for the moving part 38 which allows the moving part 38 to move between two positions, guiding means 33 for the penetrating member which allows the penetrating member to move between two positions, and guiding means 34 for the actuator handle 11 which allows the actuator handle 11 to move between two positions. The housing 30 is attached to the base part 100. This attachment is releasable.

The attachment is provided by a connection which in this embodiment comprises a hinge 35 and a locking member 14, whereby the housing 30 and base part 100 are releasably connected. The hinge 35 comprises an at least partly rounded surface of a wall of the housing 30 which can pivot in relation to the base part 100. The locking member 14 of the housing 30 interacts with locking means 108 of the base part 100.

The letter "b" in fig. 9C indicates the height of the housing 30 of the insertion device 1. The height "b" will expediently be in the range of 5-100 mm, and normally in the range 10-50 mm or more specifically 20-30 mm. The illustrated embodiment is 25 mm.

The housing 30 also comprises retention means 31. The retention means 31 hold the moving part 38 in a start position by engaging with locking means 28 on the moving part 38. According to this embodiment the retention means further provides a stop for the movement of the actuator handle 11.

The guiding means 32 for the moving part 38 provides a directional controlled movement of the moving part 38 in relation to the housing 30. The guiding means 32 are attached to or connected to or an integrated part of the inner surfaces of the housing 30 and will normally have the shape of longitudinal tracks corresponding to surfaces on the moving part 38. In the depicted embodiment, the moving part 38 can move parallel, i.e. horizontal to the base part 100, guided by the guiding means 32, the movement will normally be a sliding movement in a direction parallel to the surface of the base part 100, i.e. the movement is a longitudinal movement or a linear movement.

The guiding means 33 for the penetrating member which is a part of or connected to or integrated with the housing 30 provides that the penetrating member can only be moved in a well defined direction which direction is different from the direction of the moving member 38. In the embodiment the direction of movement of the penetrating member is essentially perpendicularly to the direction of movement of the moving part 38. The guiding means 33 for the penetrating member will normally be formed by inner surfaces of the housing 30, e.g. the guiding means 33 may comprise the inner surfaces of a hollow, cylindrical element wherein the penetrating member can move between at least a forward and a retracted position along the longitudinal axis of said cylindrical element, comparable to the movement of a piston in a cylinder. Such a movement will be a sliding movement as the continuous contact between the inner surfaces of the cylindrical elements and the outer surfaces of the penetrating member provides the guiding. Alternatively, the guiding means 33 of the penetrating member can comprise one or more bars, governing the direction of movement of the penetrating member 50. As seen from the figures the guiding means 33 for the penetrating member to this embodiment can extend from the inner ceiling of the housing to the bottom part 100. The guiding means 33 of the penetrating member not attached to the base part 100 but might reach down and touch it or e.g. provide a support for the base part 100.

The guiding means 34 of actuator handle 11 provides a directional controlled movement of the actuator handle 11 in relation to the housing 30. The guiding means 34 are attached to or integrated with the housing 30. In the depicted embodiment, the actuator handle 11 moves in parallel with, i.e. horizontal to the base part 100, guided by the guiding means 34 which according to this embodiment is provided as parts of the inner surfaces of the housing. The guiding means 34 might be formed as longitudinal tracks leading the actuator handle 11 in a well defined direction or simply the inner surfaces of the walls of the housing 30. Such a movement is normally a sliding movement as the guiding means 34 and the activation means are in continuous contact while moving in relation to each other. The movement will normally be a linear movement. The direction of movement of the .actuator handle 11 is according to this embodiment identical to the direction of movement of the moving part 38 therefore the guiding means 34 of the actuator handle 11 can be the same as the guiding means 32 of the moving part 38 i.e. on set of guiding means 32, 34 provides the well defined and at least partly simultaneous movement of the moving part 38and the actuator handle 11.

The moving part 38 is provided with transformation means 39 providing transformation of the movement of the moving part 38, which according to this embodiment is horizontal, into a movement of the penetrating member the insertion direction followed by a movement of at least the insertion needle of the penetrating member in a direction of retraction. According to this embodiment the transformation means is shaped as an open V-shaped track in the moving part 38 which corresponds to a protruding cylindrical part 51 of the penetrating member 50. The V-shaped track 39 is sized to fit closely with the protruding part 51 of the penetrating member in order to provide a well defined path for movement.

The moving part 38 comprises a releasing member 29 provides a separation of the housing 30, or at least a part of the housing 30, from the base part 100 by releasing the locking member 14 of the housing from the locking means 108 of the base part 100. Said release is provided by interaction of the releasing member 29 with a part of the housing 30, according to this embodiment it is the inner wall of the housing 30 opposite the actuator handle 11 where the linear movement of the actuator handle 11 would end if continued to the inner wall of the housing 30.

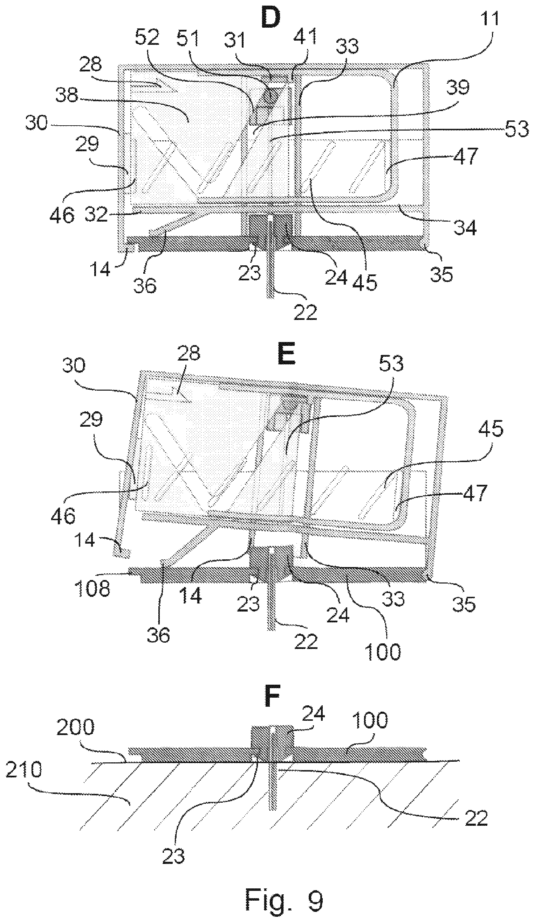

The housing comprises an elastic member 36 which upon release of the locking member 14 of the housing initiates removal of the housing 30 from the base part 100. According to the embodiment shown in fig. 9 the elastic member 36 is an integrated part of the housing 30 i.e. it is fastened unreleasably to the housing 30. The elastic member 36 is a leaf spring unreleasably fastened to the housing 30 at one end and pressed against the base part 100 at the opposite end. The flexibility of the elastic member 36 is defined by the material of which it is constructed and the physical dimensions of the material, according to the present embodiment the elastic member is constructed of the same material as the housing i.e. a hard plastic and normally formed during molding of the housing 30, but it could also be constructed of a metal which after molding of the housing is fastened unreleasably to the housing 30.

Insertion of the penetrating member using the insertion device according to the invention 1 is provided by activation of the actuator handle 11. The actuator handle 11 is activated by pushing the part towards the housing 30. The actuator handle 11 comprises interaction means 41. The interaction means 41 interacts with the retention means 31 of the housing 30, thereby arresting the forward movement of the actuator handle 11. As can be seen in fig. 9A, the actuator handle 11 protrudes the housing 30 in the depicted, non-activated state. The letter "a" indicates the length of protrusion of the actuator handle 11 with respect to the housing 30. The protrusion before activation of the actuator handle 11 will normally be in the range of 1-100 mm, or 5-50 mm, or 10-25 mm, or 15-20 mm. In the shown embodiment the protrusion is 17 mm. In another embodiment of the invention, the actuator handle 11 does not protrude the housing 30, or protrudes the housing 30 only marginally or slightly.

The insertion device 1 is normally in a non-activated state before use, such as during transport or storage.

According to this embodiment a spring 45 is provided between the moving part 38 and the actuator handle 11. Normally the spring 45 will in a relaxed state during storing as this will normally prolong the time the product can be stored while still being fully functional, if the spring 45 is in a biased state during storing there is a risk that the performance of the product will rapidly decrease. As illustrated in fig. 9A-F the spring 45 can be a spiral spring, comprising two ends: a first end 46, attached to, or placed in connection with the moving part 38 and a second end 47 attached to, or placed in connection with the actuator handle 11. The spring 45 is positioned along the direction of movement for the actuator handle 11 which is being parallel to the upper surface of the base part 100.

A function of the spring 45 is to provide energy for the penetration and/or retraction movement of the penetrating member and/or parts of the penetrating member 50. If this energy is not provided by a spring 45 it has to be directly provided by the user of the device as the user provides a horizontal movement of the actuator handle 11 by pushing the actuator handle 11 towards the housing 30 and thereby a horizontal movement of the moving part 38.

The spring 45 of the illustrated embodiment store energy from the movement of the actuation of the of the actuator handle 11 as the spring 45 is biased through this movement. During actuation of the actuator handle 11 the moving part 38 is stationary. When the interaction means 41 of the actuator handle 11 gets into contact with the locking means 28, the moving part 38 is released from the stationary position and moved in a direction defined by the guiding means 13. The forward movement of the actuator handle 11 is stopped at the time where the interaction means 41 touches the retention means 31 of the housing 30. According to the embodiment of fig. 9 the direction of the moving part 38 is the same as the forward direction of the actuator handle 11. When the moving part 38 pushed by the spring 45 hits the inner surface of the housing 30, the spring 45 is biased enough to provide energy for release of the releasable connection between the locking member 14 of the housing 30 and locking means of the base part 100. This is provided by making the wall or at least a part of the wall of the housing 30 so flexible that the wall can be bend outward and release the locking member 14 from the base part 100. When the locking connection is released the elastic member 36 pushes the housing 30 away from the base part 100 and the user need not pull the insertion device away from the base part 100.

As illustrated in fig. 9 A-F the insertion device 1 is adapted to provide:
(I) a first position (fig. 9A) where the penetrating member is fully retracted and not protruding from the insertion device 1, the actuator handle 11 protrudes the housing 30 and has not been activated/actuated, the base part has been positioned on the patients skin and the penetrating member is in this position sterile and ready for insertion;
(II) a second position (fig. 9B) where the actuator handle 11 has been actuated i.e. pushed towards the housing 30, and the locking means 28 of the moving part has been released and the interaction means 41 has been brought into contact with the retention means 31 but the penetrating member and the moving part 38 have not moved yet;
(III) a third position (fig. 9C), where the part(s) of the penetrating member which are to be inserted subcutaneously into the patient, such as a cannula 54 and/or an insertion needle 53, protrude from the housing 30 through an opening 101 of the base part 100; the body 55 of the penetrating member is attached to the base 100 with holding means in the form of mechanical hooks catching hold of and edge of the base part 100;
(IV) a fourth position (fig. 9D), where the moving part 38 has moved to its end position against the inner wall of the housing 30, the holding means of the penetrating member keeps the cannula and the cannula body of the penetrating member in position while the insertion needle 53 has been retracted to such an extend that it is fully freed and has no more contact with the parts of the penetrating member remaining in inserted position, and the housing 30 and the base part 100 are still connected by the locking member 14;
(V) a fifth position (fig. 9E); where the pushing of the spring 45 against the inner wall of the housing 30 has released the locking member 14 and the housing 30 is pivoted around the hinge 35 as the elastic member 36 pushes the housing 30 away from the base part 100; and
(VI) a sixth position (fig. 9F), where the housing 30 is completely detached from the base part 100 leaving the base part 100 attached to the surface of the skin 200 of a patient, and the cannula 54 inserted into the patients tissue 210.

Fig. 10 shows an enlargement of the cannula part 7 shown in fig. 1. This embodiment comprises a body 24 provided with a cannula 22 and with a protruding front 25 having a flat surface provided with an opening 20. The protruding front 25 of the cannula part 7 need not be flat; it can actually have any desired shape as long as it is possible to create a corresponding surface on the connection part 3 facing the cannula part 7. In one embodiment the front 25 is inclined in such a way that the cross-section at the upper i.e. distal end of the cannula part 7 is larger than the cross-section at the proximal end of the front, i.e. the end closest to the patient after insertion. The opening 20 of the protruding front 25 is an inlet or outlet through which liquid can enter or exit the cannula part 7. The body 24 is further provided with a top opening 21 which can be covered with a self closing membrane. The opening 21 need some kind of entrance protection as it is facing an outer surface which is in contact with the surroundings. The top opening 21 is primarily used when inserting the cannula part 7 if the cannula 22 is a soft cannula. That the cannula 22 is soft means that it is made of a relatively soft material which can not by it self penetrate the patients skin, in this case it is necessary to use a pointy insertion needle of a relatively hard material when inserting the cannula and this pointy needle can be inserted through the top opening 21, pass through an inner hollow in the body 24 of the cannula part and further pass through the full length of the cannula 22 in such a way that the pointy end of the insertion needle stick out of the open end of the hollow cannula 22. After insertion i.e. after the cannula 22 has been placed sub- or transcutaneous in the patient, then the insertion needle is retracted and the cannula 22 is left inside the patient. The cannula part 7 is also provided with fastening means 23 which in this embodiment has the form of a series of outward hooks 23 which are flexibly fastened to the body 24 in such a way that the hooks can pivot inwards toward the centre of the cannula part 7. When the cannula part 7 is pressed toward the base part, the hooks 23 passes an edge which pushes them toward the centre as they passes the edge and when the hooks have passed the edge they return to their original position and as a upward surface of one or more of the hooks touch a downward surface of the edge the cannula part 7 is locked unreleasably against the edge.

Fig. 11 shows an enlargement of a second embodiment a cannula part 7. This embodiment also comprises a body 24 provided with a cannula 22 and with a protruding front 25 having a flat surface provided with an opening 20 but according to this embodiment the protruding front 25 is inclined in order to reduce the force required to insert the cannula part and in order to reduce distortion of the sealing 18. The inclination of the front 25 is defined by the angle d between the centre line c of the cannula 22 and a line parallel to the surface around the opening 20. The angle d will be larger than 0°and smaller than 90°, normally d ∈ ]0°, 30°] depending on the diameter of the sealing 18 or [60°, 90°[. The distance d₁ between at the distal end of the surface of the protruding part 25, i.e. the end of the cannula part 7 which is furthest away from the patient after insertion, and the centre c of the cannula part 7 is larger than the distance d₂ between the surface of the protruding part 25 at the proximal end, i.e. the end closest to the patient after insertion, and the centre c of the cannula part 7. Normally the distance d₂ will be so small that the proximal end of the protruding front 25 does not touch the sealing 18 of the connection part 3 during insertion.

In one embodiment (not shown) the angle d is close to 90° i.e. d = 90°, such an embodiment would in a drawing corresponding to fig. 11 appear to have an upward opening 12 of the connection part 3 fitting to a downward opening 20 of the cannula part 7. This means that the force pushing the cannula part 7 toward the sealing 18 will be close to perpendicular to the contact surface of the sealing 18 and this will prevent that the sealing is distorted during insertion of the cannula part 7 by the cannula part 7 sliding along the sealing 18.

In another embodiment (shown in fig. 10) d = 0° as the protruding front 25 and the centre line c are parallel. According to this embodiment the cannula part 7 will be in sliding contact with the protruding sealing 18 which can cause the sealing to be distorted.

As according to the embodiment of fig. 10 the protruding front 25 of the cannula part 7 need not be flat; it can actually have any desired shape as long as it is possible to create a corresponding surface on the connection part 3 facing the cannula part 7. Also the opening 20 of the protruding front 25 can be an inlet or an outlet depending on the purpose of the cannula part 7. In fig. 11 which is a cut-through view it is shown how the top opening 21 of the body 24 is covered with a self closing membrane 21A. As according to the embodiment of fig. 10 the top opening 21 is primarily used when inserting the cannula part 7 if the cannula 22 is a soft cannula but the top opening 21 can also be used to inject medication or nutrients other than the primary medication which could be e.g. insulin which the patient receive via the opening 20.

This embodiment of the cannula part 7 is also provided with fastening means 23 and in this embodiment the fastening means 23 has the form of a protruding part 23 on the cannula part 7 which corresponds to a flexible part 23A on the stationary base part. The flexible part 23A can be pushed outward as indicated with an arrow at fig. 11 when the protruding part 23 on the cannula part 7 passes during insertion of the cannula part 7. After insertion the upward surface of the protruding part 23 of the cannula part 7 will be locked by the downward surface of the flexible part 23A of the base part and it will not be possible to detach the cannula part 7 from the base part.

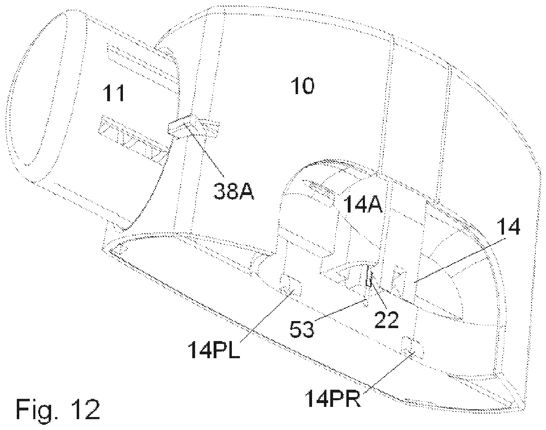

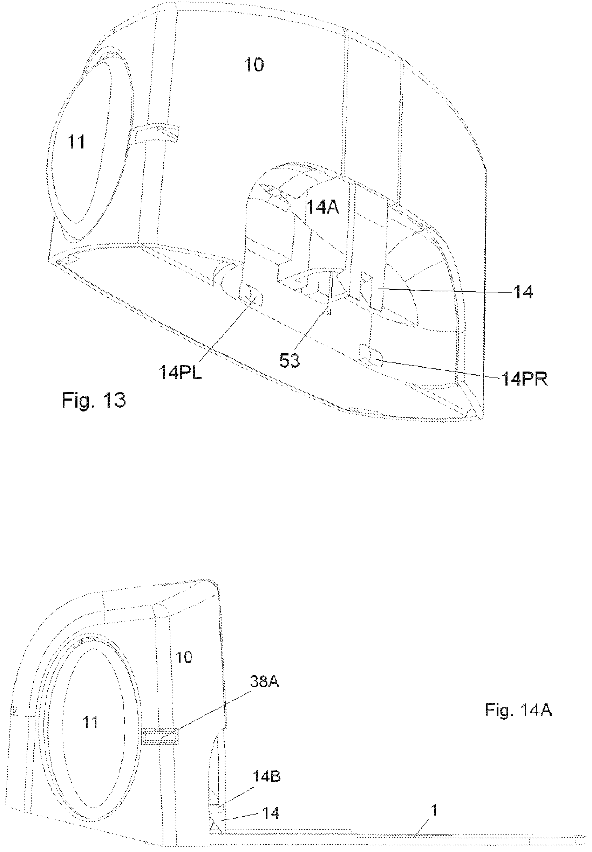

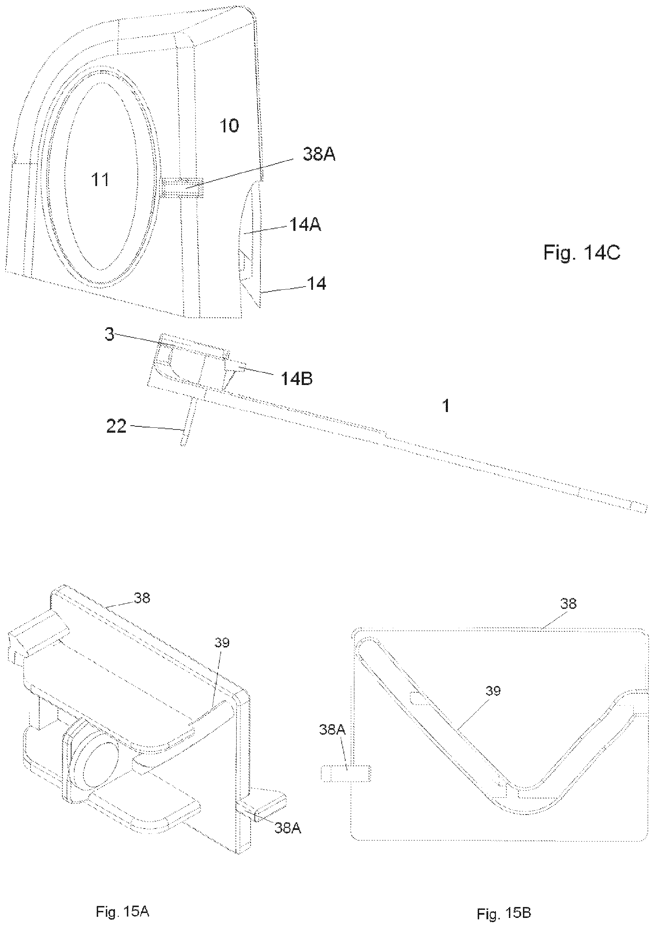

Figs. 12-14 show a third embodiment of an inserter, in fig. 12 and 13 the inserter is shown separated from the rest of the assembly. The inserter 10 comprises like the first and second embodiment of the inserter an actuator handle 11 which in fig. 12 is shown in a pre-insertion state and in fig. 13 is shown in an after-insertion state. The third embodiment of the inserter is provided with a moving part 38 as the embodiment of fig. 9 and this moving part is provided with a protruding member 38A which is an integrated part of the moving part 38. The moving part 38 is shown two different views in fig. 15A and 15B. That it is "an integrated part" means that it moves simultaneously with the moving part and is positioned stationary in relation to the moving part. Normally it will be molded together with the moving part and be of the same material, but it can also be made of a different material and attached to the moving part 38 after the moving part 38 has been produced.

The protruding part 38A on the moving part 38 is provided with a ramp. The ramp is an inclined surface placed on the forward side of the protruding part 38A in such a way that the front profile of the protruding part 38A forms an arrowhead.

The fastening means of this embodiment comprises a hinged part 14 which is fastened to the housing of the inserter 10. In the shown embodiment the hinged part 14 is actually made as a part of the housing as the hinged part 14 is created by making two cuts in the full height of the housing. The housing is normally made of a hard, molded plastic such as polypropylene and the relatively long shape of the hinged part 14 makes it very flexible i.e. the hinged part 14 is very pliant and it will be easy to push it outward from the relaxed position, the inward movement is blocked by the presence of the guiding means 33 for the penetrating member which in this embodiment is a cannula part 7. The hinged part 14 can also be made of a material which is different from the material of the housing of the inserter e.g. metal which are then attached to the housing in a rotatable manner.

The hinged part 14 is provided with two inward hooks ("inward" means that the hooks point toward the inside of the housing) at the lower or proximal end of the hinged part 14 and the two hooks lock the housing to the base part by catching a stationary protruding part 14B of the base part. As the two hooks are turned inward they are released from their locked position by being pushed outward i.e. away from the centre of the housing. The hinged part 14 is also provided with a contact member 14A having the form of a rounded plate of a rigid material placed inwards from the hinged part 14 around the guiding means 33 for the cannula part 7. When the moving part 38 moves from its start position to its end position the protruding member 38A which is placed on the trailing edge of the moving part 38 will hit the contact member 14A with the ramp surface and the contact member 14A will be forced outward and so will the hinged member 14 as the contact member 14A is attached unreleasably and rigidly to the hinged member 14.

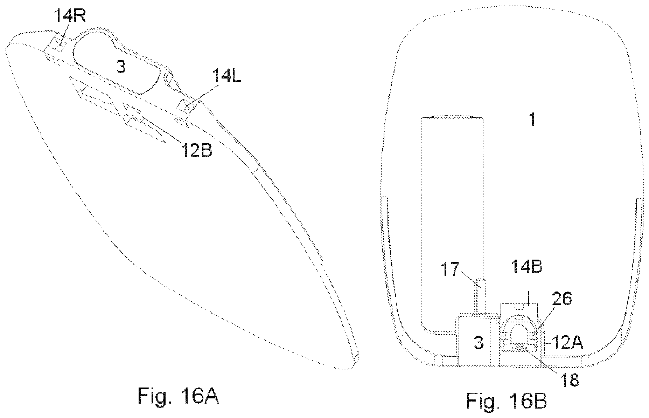

The housing of the inserter also comprises two protruding parts having the form of rounded hooks 14PL and 14PR on the inside surface of the wall opposite the inward hooks of the hinged.member 14. These protruding parts 14PL and 14PR fits into corresponding openings 14L and 14R of the base part close to the connector part 3. The openings in the base part are shown in fig. 16A. When the fastening means in the form of the openings 14L and 14R on the base part is engaged with the corresponding fastening means in the form of the rounded hooks 14PL and 14PR on the inserter 10, the inserter 10 is prevented from moving in relation to the base part, both in the direction parallel to the longitudinal direction of the base part as the protruding parts are rounded and form a grip around the opening, and also in the direction perpendicular to the surface plate 1 due to the insertion of the protruding part into the opening. After having fully inserted the penetrating member (fig. 13), the inserter 10 can be removed or detached from the base part.

In order to detach the inserter 10 from the base part, the inserter 10 is pivoted around an axis provided by the upper surface of the openings 14L and 14R. The upper (distal) surface of the openings 14L and 14R provide a contact surface for the rounded hooks 14PL and 14PR along which contact surface the downward contact surface of the rounded hooks 14PL and 14PR can slide and thereby be forced out of the openings 14L and 14R when subjecting the inserter housing 10 to a rotational movement.

The rotatable movement is made possible at the lower or proximal surface of the housing of the inserter is inclined in relation to the upper surface 1 of the base part and therefore leaves room for the displacement of the housing 10, at the end of the rotational movement the lower (proximal), inclined surface of the inserter housing will normally rest against the patients skin.

Fig. 14A shows the inserter in a position before insertion. In this state the inclined lower surface is lifted away from the patient's skin. The inward hooks of the hinged part 14 are locked around the protruding part 14B on the base part.

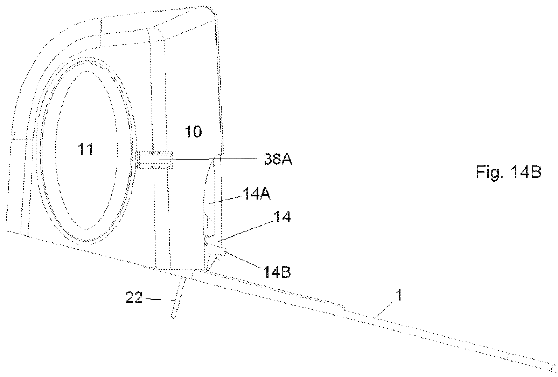

Fig. 14B shows the inserter after the cannula part has been inserted. In this state the inclined lower surface is parallel to the patient's skin and the inward hooks of the hinged part 14 have been released from the locked position.

Fig. 14C shows the inserter after it has been removed from the base part.

Fig. 15A and 15B show the moving part 38 of the third embodiment of the inserter shown in fig. 12-14. Fig. 15A shows the "back side" i.e. the side turned away from the penetrating member and fig. 15B shows the "front side" i.e. the side turned toward the penetrating member. The figures show the protruding part 38A placed at the trailing edge of the moving part 38 having the inclined side i.e. the ramp facing forward in the direction of movement, and the figures show the transformation means 39 in the shape of a longitudinal opening formed as a V where the start position is at the upper end of the first line in the V and the end position for the penetrating member is at the upper end of the second line in the V.

Fig. 16A and 16B show a base part which can be used with the third embodiment of the inserter. Fig. 16A show the openings 14L and 14R, according to this embodiment the openings are rounded in a 90 degree angle and are open toward the proximal surface of the base part i.e. the surface which is placed against the patients skin.

Fig. 16B show the base part seen from above. From this angle it is possible to see down the cannula opening 12A which according to this embodiment is provided with guiding means 26 for the cannula part 7. This guiding means 26 comprise to opposite longitudinal tracks which assure the correct placement of the cannula part 7.

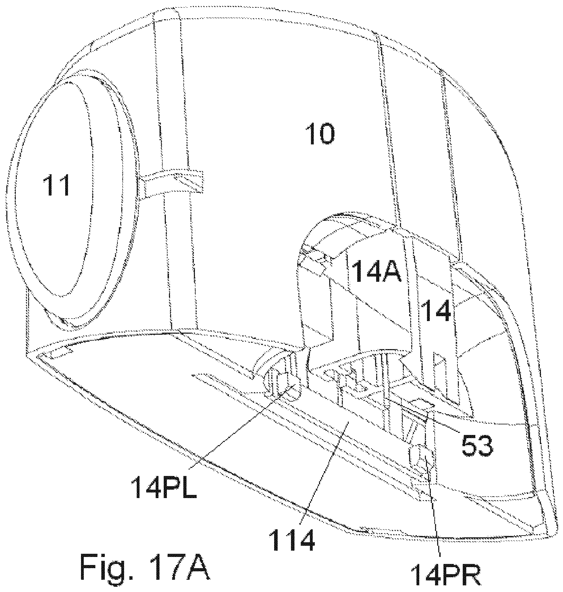

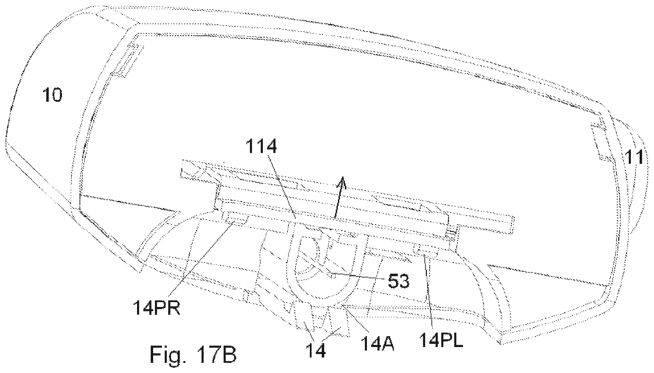

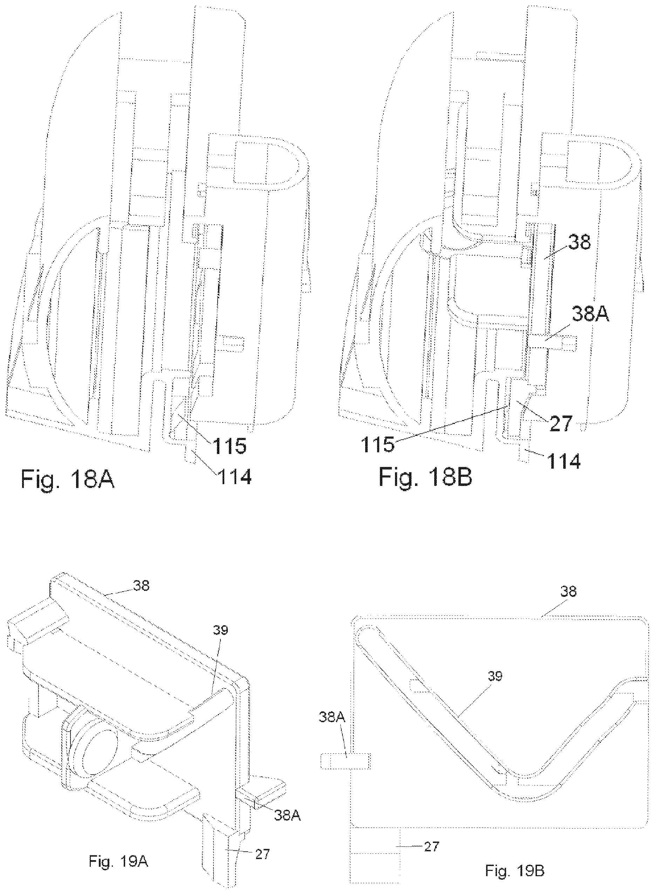

Fig. 17 and 18 show a fourth embodiment of an inserter, this embodiment differs from the third embodiment by the fastening means 14 securing the inserter to the base part. The inserter 10 is in fig. 17 and 18 shown in an after-insertion state where it has been removed from the base part. The fourth embodiment has means to release to sets of functionally independent fastening means which are supporting each other.

Like the third embodiment the fourth embodiment of the inserter is provided with a moving part 38 (see fig. 19A and 19B) having a protruding member 38A being an integrated part of the moving part 38. The moving part 38 of the fourth embodiment is further provided with a second integrated part called the positioning means 27. These positioning means 27 are attached to the lower trailing edge of the moving part 38.

The fastening means of this embodiment comprises like the third embodiment of the inserter a hinged part 14 which is fastened to the housing of the inserter 10 and the hinged part 14 moves in the same way as described for the third embodiment of fig. 12 and 13. The hinged part 14 of the fourth embodiment is also provided with two inward hooks at the lower or proximal end of the hinged part 14. The two hooks lock the housing against the base part by catching a stationary protruding part 14B of the base part having a downward or proximal contact surface. As the two hooks are turned inward they are released by being pushed outward i.e. away from the inside of the housing.

The hinged part 14 is also provided with a contact member 14A having the form of a plate placed in a direction toward the centre of the inserter i.e. "inwards" from the hinged part 14 around the guiding means 33 for the cannula part 7. When the moving part 38 moves from its start position to its end position the protruding member 38A which is placed on the trailing edge of the moving part 38 will hit the contact member 14A with the ramp surface of the protruding member 38A and the contact member 14A will be forced outward and so will the hinged member 14 as the contact member 14A is attached unreleasably and rigidly to the hinged member 14.

According to the fourth embodiment the protruding members 14PL and 14PR are positioned on a flexible member 114. The protruding members 14PL and 14PR according to this embodiment have a rectangular profile but could also have e.g. a round or triangular profile. The protruding members 14PL and 14PR fits into openings 14P and 14L of the base part close to the connector part 3. These openings correspond to the rectangular protruding members 14PL and 14PR. When the fastening means in the form of the openings 14L and 14R on the base part are engaged with the corresponding fastening means in the form of the protruding members 14PL and 14PR on the inserter 10, the inserter 10 is prevented from moving in relation to the base part, both in the direction perpendicular to the surface plate 1 and in any direction parallel to the surface plate 1.

The flexible member 114 is attached to the housing or a part being stationary in relation to the housing 10 in such a way that the flexible member can move between two positions, a first position where the inserter is locked to the base part, and a second position where the inserter is released from the base part. Both fig. 17A and 17B show the flexible member 114 in a relaxed locked position and an arrow in fig. 17B indicates the direction it moves in, in order to get to the second released position. According to the shown embodiment the flexible member 114 is made as an integrated part of the guiding means 32 for the moving part i.e. the flexible member 114 constitutes a part of the surfaces or walls along which the moving part 38 slides. The flexible member 114 is provided with a contact part 115 which according to this embodiment has a triangular profile with the sharpened edge pointing forward in the direction of movement during insertion. The contact part 115 is formed with a ramp shaped surface pointing in the direction opposite of the direction of movement of the moving part 38 during insertion.

In order to bring the flexible member 114 from a first relaxed and locked position into a second and released position the flexible has to be subjected to a force large enough to be able to move the flexible member 114 to the second position.

Fig. 18A and 18B shows the internal parts of the inserter housing 10 which provide the guiding parts for the moving part and which are not visible when the surrounding housing is in place. Fig. 18A and 18B show identical cuts through these internal housing parts but in fig. 18A the moving part 38 is removed in order to make the contact part 115 of the internal parts visible. The contact part 115 consists of a protruding ramped surface which will get in contact with the positioning part 27 of the moving part 38 when the moving part 38 is in its end or final position.

Fig. 19A and 19B show the moving part 38 of the fourth embodiment of the inserter shown in fig. 17-18. Fig. 19A shows the "back side" i.e. the side turned away from the penetrating member and fig. 19B shows the "front side" i.e. the side turned toward the penetrating member. The figures show the protruding part 38A placed at the trailing edge of the moving part 38 having the inclined side i.e. the ramp facing forward in the direction of movement, and the figures show the transformation means 39 in the shape of a longitudinal opening formed as a V where the start position is at the upper end of the first line in the V and the end position for the penetrating member is at the upper end of the second line in the V. The end position is placed lower than the start position. At the lower edge of the moving part 38 is shown positioning means 27 which assures the positioning of the moving part 38 in relation to the housing of the inserter when sliding along the guiding means 32 provided by the surrounding parts of the inserter housing but which main function is to force the flexible member 114 of the housing "backwards" when the moving part 38 and the integrated positioning means 27 passes by.

When the positioning means 27 of the moving part 38 touch the flexible member 114, the flexible member 114 is pushed away from the connection part 3 of the base part, and the fastening means in the form of the protruding parts 14PL and 14PR are pulled out of the corresponding openings in the base part 14L and 14R. When the moving part 38 is in its end position, the integrated parts 38A and 27will be at positions where both the hinge part 14 and the flexible member are pushed away from their relaxed and locked position which means it will be possible to remove the inserter from the base part when the moving part 38 is in its end position.

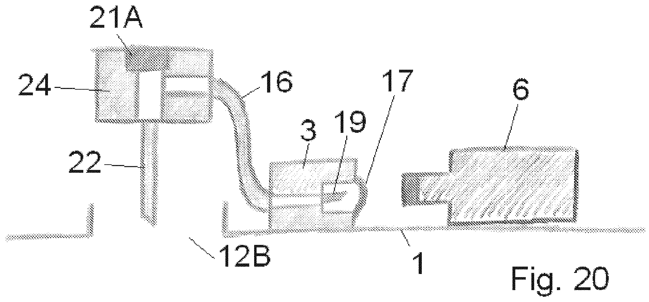

Fig. 20 shows part of an embodiment of an assembly according to the invention. The figure shows the surface plate 1, a penetrating member in the form of a cannula part 7 having a body 24, a cannula 22 and a self closing membrane 21A protecting an upper access of the body 24. The cannula part 7 has a further access opening 20 which is connected to a connection part 3 via a tube shaped connection 16 which connection provides a permanent fluid path between the cannula part 7 and the connection part 3 although the cannula part 7 is not yet placed in a in-use-position. A reservoir containing medication is also shown in a position where it could be situated before it is joined to the connection part 3 via the connector needle 19 and forms a fluid path with the cannula part.

The flexible connection 16 either has to be long and flexible enough to reach between the cannula part and the connection part 3 in both a retracted position and a forward position or the flexible connection 16 has to be elastic and able to be extended in order to let the cannula part get into the retracted position. When the cannula part is in the retracted position it is placed inside an inserter (not shown), it could be an inserter of the type shown in any of the previous figures or it could be a completely different inserter which have the ability to hold the penetrating member to be inserted.

The flexible connection 16 attaches the penetrating member 7 unreleasably to the surface plate 1.

| **Ref No** | **Name** |
|---|---|
| 1 | Surface plate |
| 2 | Mounting pad |
| 3 | Connection part |
| 4 | Guiding means |
| 5 | Magnet |
| 6 | Reservoir |
| 7 | Cannula part |
| 8 | Delivery part |
| 9 | Release handles of delivery part |
| 10 | Inserter |
| 11 | Actuator handle for inserter |
| 12 | Outlet or Second opening |
| 12A | Cannula opening |
| 12B | Opening in surface plate for cannula part |
| 13 | Inlet or First opening |
| 14 | Fastening means for inserter / hinged part |
| 14A | Protruding member on locking means (fig. 12-14) |
| 14B | Part on base part corresponding to fastening means for inserter |
| 14R | Fastening means for inserter (Right side) |
| 14L | Fastening means for inserter (Left side) |
| 14PR | Protruding fastening means of inserter (Right side) |
| 14PL | Protruding fastening means of inserter (Left side) |
| 15 | Protruding parts of base part |
| 16 | Flexible connection between cannula part and base part |
| 17 | Bubble membrane |
| 18 | Sealing around outlet or second opening |
| 19 | Connector needle |
| 20 | Opening into cannula part |
| 21 | Top opening in cannula part |
| 21A | Self-closing membrane |
| 22 | Cannula |
| 23 | Fastening means |
| 24 | Body of cannula part |
| 25 | Protruding front |
| 26 | Guiding means for cannula part in cannula opening 12A |
| 27 | Positioning means |
| 28 | Locking means |
| 29 | Releasing member |
| 30 | Housing |
| 31 | Retention means |
| 32 | Guiding means for movinq part |
| 33 | Guiding means for penetrating member |
| 34 | Guiding means for activation part |
| 35 | Hinge |
| 36 | Elastic member |
| 37 | - |
| 38 | Moving part |
| 38A | Protruding part on moving part (fig. 12-14) |
| 39 | Transformation means |
| 40 | |
| 41 | Interaction means |
| 45 | Spring |
| 46 | First end of spring in connection with moving part |
| 47 | Second end of spring in connection with activation part |
| | |
| 50 | Penetrating member |
| 51 | Transformation means |
| 52 | Holding means holding penetrating member |
| 53 | Insertion needle |
| | |
| 100 | Base part |
| | |
| 114 | Flexible member of inserter housing |
| 115 | Contact part of flexible member |
| | |
| | |
| 200 | Skin surface |
| 210 | Tissue |

## Claims

1. An assembly comprising
- an inserter (10) for insertion of a penetrating member (7) into a patient,
- a penetrating member (7) attached to the inserter (10) before use which penetrating member (7) is provided with a through going opening forming a fluid connection between delivery means (8) and a patient when the penetrating member (7) is inserted,
- a base part comprises a position shaped to receive the penetrating member (7) where the penetrating member (7) can be attached to the base part during use and fastening means (2) which can provide for the base part to be attached to the patient,
where the inserter (10) comprises inserter attachment means (14) locking the inserter (10) to the base part before and during insertion of the penetrating member (7) which inserter attachment means (14) can be unlocked so that the inserter can be removed from the base part after insertion of the penetrating member (7), the inserter attachment means (14, 14A, 14B, 14PR/R, 14PL/L) comprise at least one protruding part and at least one corresponding opening,
**characterized in that** either the at least one protruding part or the at least one corresponding opening part is positioned on a surface of the base part and a corresponding part either comprising a protruding part or an opening is positioned on a surface of the inserter (10) and that either the penetrating member (7) or the base part or both is/are provided with sealing means (18) forming a fluid tight connection between the base part and the penetrating member (7) and the base part is further provided with sealing means (17) forming a fluid connection to delivery means (8).

2. An assembly according to claim 1, **characterized in that** the inserter (10) is released from the base part i.e. the inserter attachment means (14) are disengaged, by the interaction between a moving part (38) of the inserter (10) which moving part guides the insertion of the penetrating member (7) from a first to a final position and a stationary part (29, 115, 14A) of the base part during insertion.

3. An assembly according to claim 2, **characterized in that** the moving part (38) of the inserter (10) moves forward in a direction which is approximately perpendicular i.e. 90°±45°, normally 90°±15° to the direction of insertion of the penetrating member (7).

4. An assembly according to any preceding claim, **characterized in that** the penetrating member (7) is attached by a tube shaped connection (16) to the base part before and during insertion.

5. An assembly according to claim 4, **characterized in that** the connection (16) comprises a flexible material.

6. An assembly according to any preceding claim, **characterized in that** the inserter (10) is released from the base part by applying a force to the inserter (10) or a part of the inserter (10) in a direction different from the direction of insertion of the penetrating member (7).

7. An assembly according to claim 5, **characterized in that** the force is applied in a direction which is approximately perpendicular i.e. 90°±45°, normally 90°±15° to the direction of insertion of the penetrating member (7).

8. An assembly according to any preceding claim, **characterized in that** the release of the inserter (10) from the base part is at least partly provided by the release of a force in direction towards the base part.

9. An assembly according to claim 8, **characterized in that** the released force is provided by a spring unit (45) exercising a force directed to a proximal surface of the inserter housing and a distal surface of the base part (1).

10. An assembly according to claim 9, **characterized in that** the spring (36) is a leaf spring which at one end is fastened unreleasably to a part of the inserter and at the other end will touch the upper surface of the base part before the inserter is released from the base part.

11. An assembly according to any of the preceding claims, **characterized in that** the attachment means (14) of the inserter (10) comprise at least one protruding part (14PR, 14PL) corresponding to an opening (14R, 14L) in the base part.

12. An assembly according to claim 11, **characterized in that** the at least one protruding part (14PR, 14PL) is inserted into the corresponding opening (14R, 14L) in a direction approximately parallel to the upper surface of the base part.

13. An assembly according to any of the preceding claims, **characterized in that** the attachment means (14) comprise at least one hook attached to a hinged part (14) formed by a part of the housing of the inserter (10).

14. An assembly according to claim 13, **characterized in that** the at least one hook is turned inward i.e. the hook is placed on an inner surface of the housing of the inserter and turned toward the centre of the housing.

15. An assembly according to claim 14, **characterized in that** the at least one hook faces at least two protruding parts (14PR, 14PL) which are placed on a surface of the housing opposite the at least one hook.

16. An assembly according to claim 15, **characterized in that** the at least one hook is placed between the two protruding parts.

17. An assembly according to any of the preceding claims, **characterized in that** the penetrating member (7) is provided with attachment means (23) assuring that the penetrating member (7) is unreleasably attached to the base part after insertion.

18. An assembly according to any of the preceding claims, **characterized in that** the position shaped to receive the penetrating member (7) is formed as a longitudinal opening (12A) extending from the upper surface of the connection part (3) of the base part to the lower surface of the connection part (3) of the base part, which opening (12A) corresponds to the profile of at least the non-penetrating end of the penetrating member (7).

19. An assembly according to claim 18, **characterized in that** an outlet of a fluid path (12) opens into the wall of the opening (12A) and when the penetrating member (7) is inserted, an inlet to a fluid path of the penetrating member (7) corresponds to the outlet (12) of the fluid path.

20. An assembly according to any of the preceding claims, **characterized in that** one penetrating member (7) comprises a cannula part having a body made of a hard material to which a soft or cutting cannula (22) is unreleasably attached.

21. An assembly according to claim 20, **characterized in that** the body (24) of the cannula part has an inner opening from which inner opening fluid is led to the cannula (22) and this inner opening has at least one inlet (20, 21) through which fluid or a needle can enter the inner opening.

22. An assembly according to any of the preceding claims, **characterized in that** at least one penetrating member (7) comprises a sensor part.

23. An assembly according to any of the preceding claims, **characterized in that** the assembly is delivered to the user in a sterile state where the penetrating member (7) is in a retracted position in the inserter (10) and the inserter (10) is attached to the base part.

## Patentansprüche

1. Anordnung, umfassend
- ein Einführinstrument (10) für die Einführung eines eindringenden Elements (7) in einen Patienten,
- ein eindringendes Element (7), das am Einführinstrument (10) vor der Verwendung angebracht wird, wobei das eindringende Element (7) mit einer durchgehenden Öffnung bereitgestellt ist, die eine Fluidverbindung zwischen Abgabemitteln (8) und einem Patienten bildet, wenn das eindringende Element (7) eingeführt ist,
- ein Bodenteil, das eine Position umfasst, die geformt ist, um das eindringende Element (7) aufzunehmen, wobei das eindringende Element (7) am Bodenteil während der Verwendung angebracht werden kann, und Befestigungsmittel (2), die dafür sorgen, dass das Bodenteil am Patienten angebracht wird,
wobei das Einführinstrument (10) Anbringungsmittel (14) für das Einführinstrument umfasst, die das Einführinstrument (10) am Bodenteil vor und während der Einführung des eindringenden Elements (7) verriegeln, wobei das Anbringungsmittel (14) für das Einführinstrument entriegelt werden kann, so dass das Einführinstrument aus dem Bodenteil nach der Einführung des eindringenden Elements (7) entnommen werden kann, wobei die Anbringungsmittel für das Einführinstrument (14, 14A, 14B, 14PR/R, 14PL/L) mindestens ein vorstehendes Teil und mindestens eine entsprechende Öffnung umfassen,
**dadurch gekennzeichnet, dass** entweder das mindestens eine vorstehende Teil oder das mindestens eine entsprechende Öffnungsteil auf einer Fläche des Bodenteils positioniert ist, und ein entsprechendes Teil, das entweder ein vorstehendes Teil oder eine Öffnung umfasst, an einer Fläche des Einführinstruments (10) positioniert ist, und dass entweder das eindringende Element (7) oder das Bodenteil oder beide mit Dichtungsmitteln (18) bereitgestellt ist/sind, die eine flüssigkeitsdichte Verbindung zwischen dem Bodenteil und dem eindringenden Element (7) bilden, und das Bodenteil weiter mit Dichtungsmitteln(17) bereitgestellt ist, die eine Fluidverbindung zu Abgabemitteln (8) bilden.

2. Anordnung nach Anspruch 1, **dadurch gekennzeichnet, dass** das Einführinstrument (10) vom Bodenteil durch Interaktion zwischen einem beweglichen Teil (38) des Einführinstruments (10) und einem stationären Teil (29, 115, 14A) des Bodenteils während der Einführung freigegeben wird, d. h. dass die Anbringungsmittel (14) für das Einführinstrument getrennt werden, wobei das bewegliche Teil die Einführung des eindringendes Elements (7) von einer ersten zu einer finalen Position führt.

3. Anordnung nach Anspruch 2, **dadurch gekennzeichnet, dass** sich das bewegliche Teil (38) des Einführinstruments (10) in eine Richtung vorwärts bewegt, die ungefähr senkrecht, d. h. 90° ±45°, normalerweise 90° ±15° zur Richtung der Einführung des eindringenden Elements (7) ist.

4. Anordnung nach einem der vorstehenden Ansprüche, **dadurch gekennzeichnet, dass** das eindringende Element (7) am Bodenteil vor und während der Einführung durch eine schlauchförmige Verbindung (16) angebracht ist.

5. Anordnung nach Anspruch 4, **dadurch gekennzeichnet, dass** die Verbindung (16) ein flexibles Material umfasst.

6. Anordnung nach einem der vorstehenden Ansprüche, **dadurch gekennzeichnet, dass** das Einführinstrument (10) vom Bodenteil freigegeben wird durch Anwenden einer Kraft auf das Einführinstrument (10) oder ein Teil des Einführinstruments (10) in eine andere Richtung als die Richtung der Einführung des eindringenden Elements (7).

7. Anordnung nach Anspruch 5, **dadurch gekennzeichnet, dass** die Kraft in eine Richtung angewendet wird, die ungefähr senkrecht, d. h. 90° ±45°, normalerweise 90° ±15° zur Richtung der Einführung des eindringenden Elements (7) ist.

8. Anordnung nach einem der vorstehenden Ansprüche, **dadurch gekennzeichnet, dass** das Freigeben des Einführinstruments (10) vom Bodenteil mindestens teilweise durch die Freigabe einer Kraft in Richtung zum Bodenteil bereitgestellt wird.

9. Anordnung nach Anspruch 8, **dadurch gekennzeichnet, dass** die freigegebene Kraft von einer Federeinheit (45) bereitgestellt wird, die eine Kraft ausübt, die auf eine proximale Fläche des Gehäuses des Einführinstruments und eine distale Fläche des Bodenteils (1) gerichtet ist.

10. Anordnung nach Anspruch 9, **dadurch gekennzeichnet, dass** die Feder (36) eine Blattfeder ist, die an einem Ende unlösbar an einem Teil des Einführinstruments befestigt ist und am anderen Ende die obere Fläche des Bodenteils berühren wird, bevor das Einführinstrument vom Bodenteil freigegeben wird.

11. Anordnung nach einem der vorstehenden Ansprüche, **dadurch gekennzeichnet, dass** die Anbringungsmittel (14) des Einführinstruments (10) mindestens ein vorstehendes Teil (14PR, 14PL) umfassen, das einer Öffnung (14R, 14L) im Bodenteil entspricht.

12. Anordnung nach Anspruch 11, **dadurch gekennzeichnet, dass** das mindestens eine vorstehende Teil (14PR, 14PL) in die entsprechende Öffnung (14R, 14L) in eine Richtung eingeführt ist, die ungefähr parallel zur oberen Fläche des Bodenteils ist.

13. Anordnung nach einem der vorstehenden Ansprüche, **dadurch gekennzeichnet, dass** die Anbringungsmittel (14) mindestens einen Haken umfassen, der an einem klappbaren Teil (14) angebracht ist, der von einem Teil des Gehäuses des Einführinstruments (10) gebildet ist.

14. Anordnung nach Anspruch 13, **dadurch gekennzeichnet, dass** der mindestens eine Haken nach innen gedreht ist, d. h. dass der Haken an einer inneren Fläche des Gehäuses des Einführinstruments positioniert ist und in Richtung zur Mitte Gehäuses gedreht ist.

15. Anordnung nach Anspruch 14, **dadurch gekennzeichnet, dass** der mindestens eine Haken mindestens zu zwei vorstehenden Teilen (14PR, 14PL) zeigt, die an einer Fläche des Gehäuses gegenüber dem mindestens einem Haken positioniert sind.

16. Anordnung nach Anspruch 15, **dadurch gekennzeichnet, dass** der mindestens eine Haken zwischen den zwei vorstehenden Teilen positioniert ist.

17. Anordnung nach einem der vorstehenden Ansprüche, **dadurch gekennzeichnet, dass** das eindringende Element (7) mit Anbringungsmitteln (23) bereitgestellt ist, die sicherstellen, dass das eindringende Element (7) nach der Einführung unlösbar am Bodenteil angebracht ist.

18. Anordnung nach einem der vorstehenden Ansprüche, **dadurch gekennzeichnet, dass** die Position, die geformt ist, um das eindringendes Element (7) aufzunehmen, als eine Längsöffnung (12A) geformt ist, die sich von der oberen Fläche des Verbindungsteils (3) des Bodenteils zur unteren Fläche des Verbindungsteils (3) des Bodenteils erstreckt, wobei die Öffnung (12A) dem Profil von mindestens dem nicht eindringenden Ende des eindringenden Elements (7) entspricht.

19. Anordnung nach Anspruch 18, **dadurch gekennzeichnet, dass** sich ein Auslauf eines Fluidwegs (12) in die Wand der Öffnung (12A) öffnet, und, wenn das eindringende Element (7) eingeführt ist, ein Einlauf in einen Fluidweg des eindringenden Elements (7) dem Auslauf (12) des Fluidwegs entspricht.

20. Anordnung nach einem der vorstehenden Ansprüche, **dadurch gekennzeichnet, dass** ein eindringendes Element (7) ein Kanülenteil umfasst, das einen Körper aufweist, der aus einem harten Material gefertigt ist, an dem eine weiche oder schneidende Kanüle (22) unlösbar angebracht ist.

21. Anordnung nach Anspruch 20, **dadurch gekennzeichnet, dass** der Körper (24) des Kanülenteils eine innere Öffnung aufweist, wobei aus der inneren Öffnung Fluid zur Kanüle (22) gelenkt wird und diese innere Öffnung mindestens einen Einlass (20, 21) aufweist, durch den Fluid oder eine Nadel in die innere Öffnung eintreten kann.

22. Anordnung nach einem der vorstehenden Ansprüche, **dadurch gekennzeichnet, dass** mindestens ein eindringendes Element (7) einen Sensorteil umfasst.

23. Anordnung nach einem der vorstehenden Ansprüche, **dadurch gekennzeichnet, dass** die Anordnung an den Benutzer in einem sterilen Zustand abgegeben wird, wobei sich das eindringende Element (7) einer eingezogenen Position im Einführinstrument (10) befindet und das Einführinstrument (10) am Bodenteil angebracht ist.

## Revendications

1. Ensemble comprenant
- un dispositif d'insertion (10) pour l'insertion d'un élément pénétrant (7) dans un patient,
- un élément pénétrant (7) fixé au dispositif d'insertion (10) avant utilisation, lequel élément pénétrant (7) est pourvu d'une ouverture traversante formant une connexion fluidique entre des moyens de délivrance (8) et un patient lorsque l'élément pénétrant (7) est inséré,
- une partie de base comprend une position mise en forme pour recevoir l'élément pénétrant (7) où l'élément pénétrant (7) peut être attaché à la partie de base en utilisation et des moyens de fixation (2) permettant de fixer la partie de base au patient,
où le dispositif d'insertion (10) comprend des moyens de fixation de dispositif d'insertion (14) bloquant le dispositif d'insertion (10) sur la partie de base avant et pendant l'insertion de l'élément pénétrant (7), lesquels moyens de fixation de dispositif d'insertion (14) peuvent être déverrouillés de sorte que le dispositif d'insertion peut être retiré de la partie de base après insertion de l'élément pénétrant (7), les moyens de fixation de dispositif d'insertion (14, 14A, 14B, 14PR/R, 14PL/L) comprennent au moins une partie en saillie et au moins une ouverture correspondante,
**caractérisé en ce que** la au moins une partie en saillie ou la au moins une partie d'ouverture correspondante est positionnée sur une surface de la partie de base et une partie correspondante comprenant une partie en saillie ou une ouverture est positionnée sur une surface du dispositif d'introduction (10) et **en ce que** l'élément pénétrant (7) ou la partie de base ou les deux sont munis de moyens d'étanchéité (18) formant une connexion étanche aux fluides entre la partie de base et l'élément pénétrant (7) et la partie de base est en outre munie de moyens d'étanchéité (17) formant une connexion fluidique avec des moyens de distribution (8).

2. Ensemble selon la revendication 1, **caractérisé en ce que** le dispositif d'insertion (10) est libéré de la partie de base, c'est-à-dire que les moyens de fixation de dispositif d'insertion (14) sont désengagés, par interaction entre une partie mobile (38) du dispositif d'insertion (10), laquelle partie mobile guide l'insertion de l'élément pénétrant (7) d'une première position à une position finale, et une partie fixe (29, 115, 14A) de la partie de base pendant l'insertion.

3. Ensemble selon la revendication 2, **caractérisé en ce que** la partie mobile (38) du dispositif d'insertion (10) avance dans une direction qui est approximativement perpendiculaire, à savoir à 90° ± 45°, normalement à 90° ± 15° par rapport à la direction d'insertion de l'élément pénétrant (7).

4. Ensemble selon l'une quelconque des revendications précédentes, **caractérisé en ce que** l'élément pénétrant (7) est fixé par une connexion en forme de tube (16) à la partie de base avant et pendant l'insertion.

5. Ensemble selon la revendication 4, **caractérisé en ce que** la connexion (16) comprend un matériau flexible.

6. Ensemble selon l'une quelconque des revendications précédentes, **caractérisé en ce que** le dispositif d'insertion (10) est libérée de la partie de base par application d'une force au dispositif d'insertion (10) ou à une partie du dispositif d'insertion (10) dans une direction différente de la direction d'insertion de l'élément pénétrant (7).

7. Ensemble selon la revendication 5, **caractérisé en ce que** la force est appliquée dans une direction approximativement perpendiculaire, c'est-à-dire à 90° ± 45°, normalement à 90° ± 15° par rapport à la direction d'insertion de l'élément pénétrant (7).

8. Ensemble selon l'une quelconque des revendications précédentes, **caractérisé en ce que** la libération du dispositif d'insertion (10) à partir de la partie de base est fournie au moins en partie par la libération d'une force dans une direction vers la partie de base.

9. Ensemble selon la revendication 8, **caractérisé en ce que** la force libérée est fournie par une unité à ressort (45) exerçant une force dirigée vers une surface proximale du boîtier de dispositif d'insertion et une surface distale de la partie de base (1).

10. Ensemble selon la revendication 9, **caractérisé en ce que** le ressort (36) est un ressort à lame qui, à une première extrémité, est fixé de manière non libérable à une partie du dispositif d'insertion et à l'autre extrémité touche la surface supérieure de la partie de base avant que le dispositif d'insertion soit libéré de la partie de base.

11. Ensemble selon l'une quelconque des revendications précédentes, **caractérisé en ce que** les moyens de fixation (14) du dispositif d'insertion (10) comprennent au moins une partie en saillie (14PR, 14PL) correspondant à une ouverture (14R, 14L) dans la partie de base.

12. Ensemble selon la revendication 11, **caractérisé en ce que** la au moins une partie en saillie (14PR, 14PL) est insérée dans l'ouverture correspondante (14R, 14L) dans une direction approximativement parallèle à la surface supérieure de la partie de base.

13. Ensemble selon l'une quelconque des revendications précédentes, **caractérisé en ce que** les moyens de fixation (14) comprennent au moins un crochet fixé à une partie articulée (14) formée par une partie du boîtier de dispositif d'insertion (10).

14. Ensemble selon la revendication 13, **caractérisé en ce que** le au moins un crochet est tourné vers l'intérieur, c'est-à-dire que le crochet est placé sur une surface intérieure du boîtier du dispositif d'insertion et tourné vers le centre du boîtier.

15. Ensemble selon la revendication 14, **caractérisé en ce que** le au moins un crochet fait face à au moins deux parties en saillie (14PR, 14PL) qui sont placées sur une surface du boîtier opposée au au moins un crochet.

16. Ensemble selon la revendication 15, **caractérisé en ce que** le au moins un crochet est placé entre les deux parties en saillie.

17. Ensemble selon l'une quelconque des revendications précédentes, **caractérisé en ce que** l'élément pénétrant (7) est pourvu de moyens de fixation (23) garantissant que l'élément pénétrant (7) est fixé de manière non libérable à la partie de base après insertion.

18. Ensemble selon l'une quelconque des revendications précédentes, **caractérisé en ce que** la position mise en forme pour recevoir l'élément pénétrant (7) est formée comme une ouverture longitudinale (12A) s'étendant à partir de la surface supérieure de la partie de connexion (3) de la partie de base à la surface inférieure de la partie de connexion (3) de la partie de base, laquelle ouverture (12A) correspond au profil d'au moins l'extrémité non pénétrante de l'élément pénétrant (7).

19. Ensemble selon la revendication 18, **caractérisé en ce qu'**une sortie d'un trajet de fluide (12) débouche dans la paroi de l'ouverture (12A) et lorsque l'entrée de l'élément pénétrant (7) est inséré, une entrée vers un trajet de fluide de l'élément pénétrant (7) correspond à la sortie (12) du trajet de fluide.

20. Ensemble selon l'une quelconque des revendications précédentes, **caractérisé en ce qu'**un élément pénétrant (7) comprend une partie de canule ayant un corps constitué d'un matériau dur auquel une canule souple ou tranchante (22) est fixée de manière non libérable.

21. Ensemble selon la revendication 20, **caractérisé en ce que** le corps (24) de la partie de canule comporte une ouverture intérieure à partir de laquelle un fluide d'ouverture intérieure est amené à la canule (22) et cette ouverture intérieure présente au moins une entrée (20, 21) à travers lequel un fluide ou une aiguille peut pénétrer dans l'ouverture intérieure.

22. Ensemble selon l'une quelconque des revendications précédentes, **caractérisé en ce qu'**au moins un élément pénétrant (7) comprend une partie de capteur.

23. Ensemble selon l'une quelconque des revendications précédentes, **caractérisé en ce que** l'ensemble est délivré à l'utilisateur dans un état stérile où l'élément pénétrant (7) est dans une position rétractée dans le dispositif d'insertion (10) et le dispositif d'insertion (10) est fixé à la partie de base.
